(19) Europäisches Patentamt
European Patent Office
Office européen des brevets

(11) **EP 1 387 823 B1**

(12) **EUROPÄISCHE PATENTSCHRIFT**

(45) Veröffentlichungstag und Bekanntmachung des Hinweises auf die Patenterteilung:
**29.12.2010 Patentblatt 2010/52**

(21) Anmeldenummer: **02740550.5**

(22) Anmeldetag: **02.05.2002**

(51) Int Cl.:
*C07C 51/215* (2006.01)    *C07C 57/04* (2006.01)

(86) Internationale Anmeldenummer:
**PCT/EP2002/004794**

(87) Internationale Veröffentlichungsnummer:
**WO 2002/090308 (14.11.2002 Gazette 2002/46)**

(54) **VERFAHREN ZUR HERSTELLUNG VON ACRYLSÄURE DURCH HETEROGEN KATALYSIERTE PARTIALOXIDATION VON PROPAN**

METHOD FOR THE PRODUCTION OF ACRYLIC ACID BY HETEROGENEOUSLY CATALYZED PARTIAL PROPANE OXIDATION

PROCEDE DE PRODUCTION D'ACIDE ACRYLIQUE PAR OXYDATION PARTIELLE DE PROPANE CATALYSEE DE FACON HETEROGENE

(84) Benannte Vertragsstaaten:
**AT BE CH CY DE DK ES FI FR GB GR IE IT LI LU MC NL PT SE TR**

(30) Priorität: **07.05.2001 DE 10122027**

(43) Veröffentlichungstag der Anmeldung:
**11.02.2004 Patentblatt 2004/07**

(73) Patentinhaber: **BASF SE**
**67056 Ludwigshafen (DE)**

(72) Erfinder: **BORGMEIER, Frieder**
**68163 Mannheim (DE)**

(56) Entgegenhaltungen:
**EP-A- 0 361 372    EP-A- 0 608 838**

# EP 1 387 823 B1

**Beschreibung**

Vorliegende Erfindung betrifft ein Verfahren zur Herstellung von Acrylsäure durch heterogen katalysierte Partialoxidation von Propan in der Gasphase, bei dem man ein Propan, molekularen Sauerstoff und wenigstens ein Verdünnungsgas enthaltendes Reaktionsgasausgangsgemisch bei erhöhter Temperatur über eine Multimetalloxidmasse der allgemeinen Stöchiometrie I

$$Mo_1V_bM^1{}_cM^2{}_dO_n \qquad (I),$$

mit

M$^1$ = Te und/oder Sb,
M$^2$ = wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, B, Ce, Sn, Zn, Si und In,
b = 0,01 bis 1,
c = > 0 bis 1,
d = > 0 bis 1 und
n = eine Zahl, die durch die Wertigkeit und Häufigkeit der von Sauerstoff verschiedenen Elemente in (I) bestimmt wird,

leitet und dabei das Propan partiell zu Acrylsäure oxidiert.
Verfahren zur Herstellung von Acrylsäure, einem zur Herstellung von Polymerisaten bedeutenden Monomeren, durch heterogen katalysierte Partialoxidation von Propan an Multimetalloxidmassen der allgemeinen Stöchiometrie I sind allgemein bekannt (vgl. z.B. DE-A 10119933, DE-A 10051419, DE-A 10046672, DE-A 10033121, EP-A 1090684, EP-A 962253, EP-A 895809, DE-A 19835247, EP-A 608838, WO 00/29105 und WO 00/29106).
Gemeinsames Merkmal der im Stand der Technik gegebenen Lehren (vgl. z.B. EP-B 608838, Seite 4, Zeilen 39/40) und erzielten Ergebnisse ist, daß eine Mitverwendung signifikanter Mengen an wasserdampf im Reaktionsgasausgangsgemisch sowohl für die Katalysatoraktivität als auch für die Selektivität der Acrylsäurebildung vorteilhaft ist.
Nachteilig an dieser im Stand der Technik empfohlenen Verfahrensweise ist jedoch, daß bei den Verfahren der gasphasenkatalytisch oxidativen Herstellung von Acrylsäure keine reine Acrylsäure sondern ein Produktgasgemisch erhalten wird, von dem die Acrylsäure abgetrennt werden muß.
In typischer Weise enthält das Produktgasgemisch einer heterogen katalysierten Gasphasenpartialoxidation von Propan zu Acrylsäure neben nicht umgesetztem Propan Nebenkomponenten wie Propen, Acrolein, $CO_2$, CO, Essigsäure und Propionsäure, aber auch die als Verdünnungsgase mitverwendeten Komponenten wie z.B. Wasserdampf, von denen die Acrylsäure abgetrennt werden muß, um zum reinen Produkt zu gelangen.
Aufgrund der ausgeprägten Affinität von Acrylsäure zu Wasser (bei den in Babywindeln eingesetzten Superabsorbern handelt es sich um Polymere auf Acrylsäurebasis) ist die vorgenannte Trennaufgabe um so aufwendiger, je mehr Wasserdampf bei der Gasphasenoxidation als Verdünnungsgas mitverwendet worden ist.
Ein Beisein erhöhter Mengen an Wasserdampf fördert außerdem die unerwünschte Bildung von Propionsäure als Nebenprodukt, das sich infolge seiner Ähnlichkeit mit Acrylsäure von letzterer nur noch äußerst schwierig abtrennen läßt.
Bevorzugt wäre daher ein Verfahren der heterogen katalysierten Partialoxidation von Propan zu Acrylsäure, das der Mitverwendung von Wasserdampf als Verdünnungsgas entweder gar nicht oder nur in einem verringerten Umfang bedarf und bei dem die Katalysatoraktivität und die Selektivität der Acrylsäurebildung trotzdem zu befriedigen vermögen.
Demgemäß wurde ein Verfahren zur Herstellung von Acrylsäure durch heterogen katalysierte Partialoxidation von Propan in der Gasphase, bei dem man ein Propan, molekularen Sauerstoff und wenigstens ein Verdünnungsgas enthaltendes Reaktionsgasausgangsgemisch bei erhöhter Temperatur über eine Multimetalloxidmasse der allgemeinen Stöchiometrie I

$$Mo_1V_bM^1{}_cM^2{}_dO_n \qquad (I),$$

mit

M$^1$ = Te und/oder Sb,
M$^2$ = wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, B, Ce, Sn, Zn, Si und In,
b = 0,01 bis 1,
c = > 0 bis 1,

d = > 0 bis 1 und

n = eine Zahl, die durch die Wertigkeit und Häufigkeit der von Sauerstoff verschiedenen Elemente in (I) bestimmt wird,

leitet und dabei das Propan partiell zu Acrylsäure oxidiert, gefunden, das dadurch gekennzeichnet ist, daß die Zusammensetzung des Reaktionsgasausgangsgemisches während der Durchführung des Verfahrens wenigstens einmal so geändert wird, daß der im Reaktionsgasausgangsgemisch, bezogen auf die im Reaktionsgasausgangsgemisch enthaltene molare Menge an Propan, vor der Änderung enthaltene molare Anteil des Verdünnungsgases Wasserdampf nach der Änderung ein geringerer ist.

Grundlage der erfindungsgemäßen Verfahrensweise ist der überraschende Befund, daß Multimetalloxidmassen der allgemeinen Stöchiometrie I ihre Fähigkeit, bei der katalytischen Gasphasenoxidation von Propan zu Acrylsäure im Beisein erhöhter Mengen an Wasserdampf als Verdünnungsgas die Acrylsäure mit erhöhter Aktivität und Selektivität zu bilden in wesentlichem Umfang beibehalten, wenn man im Reaktionsgasausgangsgemisch während einer gewissen Betriebsdauer, bezogen auf im Reaktionsgasausgangsgemisch enthaltenes Propan, zunächst einen bestimmten Anteil an Wasserdampf mitverwendet und diesen Anteil anschließend verringert. D.h., auch nach der durchgeführten Verringerung des Wasserdampfanteils im Reaktionsgasausgangsgemisch verläuft die heterogen katalysierte Gasphasenoxidation des Propan bei ansonsten unveränderten Betriebsbedingungen mit einer höheren Selektivität der Acrylsäurebildung, als wenn die heterogen katalysierte Gasphasenoxidation permanent mit dem geringeren Wasserdampfanteil im Reaktionsgasausgangsgemisch durchgeführt worden wäre. Diese Aussage trifft auch dann zu, wenn man die Verringerung des Wasserdampfanteils soweit betreibt, daß das Reaktionsgasausgangsgemisch nach der erfolgten Verringerung keinen Wasserdampf mehr enthält.

Üblicherweise wird man beim erfindungsgemäßen Verfahren die Verringerung des Wasserdampfanteils wenigstens teilweise durch eine Erhöhung des Anteils der von Wasserdampf verschiedenen, beim erfindungsgemäßen Verfahren mitverwendbaren, Verdünnungsgase ausgleichen. Der Ausgleich kann aber auch unterbleiben. Solche anderen Verdünnungsgase sind insbesondere molekularer Stickstoff, Kohlenoxide wie CO und $CO_2$, aber auch Edelgase wie He oder Ar. Propan selbst kommt ebenfalls als Verdünnungsgas in Betracht. In diesem Fall wird man das Propan beim erfindungsgemäßen verfahren in einer, bezogen auf die eingesetzte Menge an molekularem Sauerstoff, überstöchiometrischen Menge einsetzen.

Üblicherweise werden Gemische der vorgenannten Verdünnungsgase angewendet. Sie verhalten sich normalerweise beim erfindungsgemäßen verfahren als inert. D.h., sie werden bei der Durchführung des erfindungsgemäßen Verfahrens chemisch im wesentlichen nicht verändert.

Selbstverständlich kann man bei der erfindungsgemäßen Verfahrensweise die molare Verringerung des Wasserdampfanteils durch eine äquivalente Erhöhung des molaren Anteils der von Wasserdampf verschiedenen inerten Verdünnungsgase auch vollständig ausgleichen oder sogar überkompensieren. Zweckmäßigerweise wird beim erfindungsgemäßen Verfahren als Wasserdampfersatz molekularer Stickstoff verwendet.

In der Regel wird das erfindungsgemäße Verfahren bei Reaktionstemperaturen von z.B. 200 bis 550°C, oder von 230 bis 480°C bzw. 300 bis 440°C durchgeführt. Selbstredend kann beim erfindungsgemäßen Verfahren die Temperatur vor der und nach der Verringerung des Wasserdampfanteils dieselbe sein. Sie kann aber auch nach der Verringerung des wasserdampfanteils niedriger oder höher als vor der Verringerung des Wasserdampfanteils sein.

Der Arbeitsdruck (absolut) kann beim erfindungsgemäßen Verfahren sowohl 1 atm, weniger als 1 atm oder mehr als 1 atm betragen. Erfindungsgemäß typische Arbeitsdrücke sind 1,5 bis 10 bar, häufig 1, 5 bis 4 bar. Der Arbeitsdruck kann während des erfindungsgemäßen Verfahrens sowohl konstant gehalten als auch variiert werden. D.h., der Arbeitsdruck kann vor der erfindungsgemäßen Verringerung des Wasserdampfanteils größer oder kleiner als danach sein.

Natürlich kann im Rahmen des erfindungsgemäßen Verfahrens die erfindungsgemäß erforderliche Änderung der Zusammensetzung des Reaktionsgasausgangsgemisches auch z.B. periodisch aufeinanderfolgend mehrfach durchgeführt werden. D.h., nach einer gewissen Betriebsdauer mit erniedrigtem Wasserdampfanteil kann der Wasserdampfanteil im Reaktionsgasausgangsgemisch für eine gewisse Zeit wieder erhöht und danach wieder gesenkt werden u.s.w..

Als Sauerstoffquelle kann für das erfindungsgemäße Verfahren sowohl reiner Sauerstoff als auch Luft oder mit Sauerstoff angereicherte bzw. abgereicherte Luft verwendet werden.

An das für das erfindungsgemäße Verfahren zu verwendende Propan werden keine besonders hohen Ansprüche in Bezug auf seine Reinheit gestellt. Auch Propen als Begleiter enthaltendes Propan kann für das erfindungsgemäße Verfahren angewendet werden. In typischer Weise bewegt sich die Zusammensetzung des Reaktionsgasausgangsgemisches für das erfindungsgemäße Verfahren innerhalb des folgenden Rahmens (molare Verhältnisse):

| Propan | : | Sauerstoff | : | $H_2O$ | : | sonstige Verdünnungsgase = |
|--------|---|-----------|---|--------|---|---------------------------|
| 1 | : | (0,1-10) | : | (0-50) | : | (0-50). |

Vorzugsweise beträgt das vorgenannte Verhältnis 1:(0,5-5):(1-30):(0-30).

Erfindungsgemäß zweckmäßig liegt das vorgenannte Verhältnis vorab der Verringerung des Wasserdampfanteils im Bereich A = 1 : (0,1-10):(0,1-50):(0-50), und nach der Verringerung des Wasserdampfanteils im Bereich B = 1 : (0,1-10):(0-30):(0-30).

Erfindungsgemäß bevorzugt umfaßt der Bereich A die molaren Verhältnisse 1 : (0,5-5):(2-30):(0-30), und der Bereich B die molaren Verhältnisse 1 : (0,5-5):(0-20):(0-30).

Die vorgenannten Bereiche gelten insbesondere dann, wenn als sonstige Verdünnungsgase überwiegend molekularer Stickstoff eingesetzt wird. Während der Anwendung des Zusammensetzungsbereiches A für das Reaktionsgasausgangsgemisch beträgt die Reaktionstemperatur beim erfindungsgemäßen Verfahren zweckmäßig 250 bis 550°C und während der Anwendung des Zusammensetzungsbereiches B für das Reaktionsgasausgangsgemisch beträgt die Reaktionstemperatur beim erfindungsgemäßen Verfahren zweckmäßig ebenfalls 250 bis 550°C.

Im übrigen kann das erfindungsgemäße Verfahren wie die im gewürdigten Stand der Technik beschriebenen Verfahrensweisen durchgeführt werden. D.h., das Katalysatorbett kann sowohl ein Festbett, ein Wanderbett oder ein Wirbelbett sein.

Dabei können die erfindungsgemäß zu verwendenden Multimetalloxidmassen I sowohl als solche (z.B. zu Pulver oder zu Splitt zerkleinert) oder auch zu Formkörpern geformt für das erfindungsgemäße Verfahren eingesetzt werden.

Die Herstellung von für das erfindungsgemäße Verfahren geeigneten Multimetalloxidmassen I kann dem eingangs gewürdigten Stand der Technik entnommen werden. Je nach angewandtem Herstellungsverfahren kann die Struktur des resultierenden Multimetalloxids eher amorph (wie z.B. in der WO 00/29105 und in der WO 00/29106 beschrieben) oder eher kristallin (wie z.B. in der EP-A 608838, der EP-A 962253, der EP-A 895809 und der DE-A 19835247 beschrieben) sein.

Die Herstellung erfolgt dabei in der Regel so, daß bei Normaldruck (1 atm) aus Quellen (Ausgangsverbindungen) der elementaren Konstituenten der Multimetalloxidmassen ein möglichst inniges, vorzugsweise feinteiliges Trockengemisch erzeugt und dieses anschließend durch thermische Behandlung unter oxidierender (z.B. Luft), reduzierender, inerter (z.B. $N_2$) oder reduzierten Druck aufweisender Atmosphäre in ein aktives Oxid überführt wird. Selbstverständlich können für das erfindungsgemäße Verfahren aber auch die auf hydrothermalem Weg hergestellten Multimetalloxidmassen I der DE-A 10033121 verwendet werden.

Die Formung der Multimetalloxidmassen I zu für das erfindungsgemäße Verfahren geeigneten Katalysatorformkörpern kann z.B. so wie in der DE-A 10118814 und in der DE-A 10119933 beschrieben erfolgen.

Erfindungsgemäß bevorzugt werden als Multimetalloxidmassen I solche eingesetzt, in denen $M^1$ = Te. Ferner sind für das erfindungsgemäße Verfahren solche Multimetalloxidmassen I günstig, bei denen $M^2$ = Nb, Ta, W und/oder Titan ist. Vorzugsweise ist $M^2$ = Nb. Der stöchiometrische Koeffizient b der erfindungsgemäß zu verwendenden Multimetalloxidaktivmassen I beträgt mit Vorteil 0,1 bis 0,6. In entsprechender Weise beläuft sich der vorzugsbereich für den stöchiometrischen Koeffizienten c auf 0,01 bis 1 bzw. auf 0,05 bis 0,4 und günstige Werte für d betragen 0,01 bis 1 bzw. 0,1 bis 0,6. Besonders günstige erfindungsgemäß zu verwendende Multimetalloxidmassen I sind solche, bei denen die stöchiometrischen Koeffizienten b, c und d simultan in den vorgenannten Vorzugsbereichen liegen. Weitere erfindungsgemäß geeignete Stöchiometrien sind jene, die in den Schriften des eingangs zitierten Standes der Technik offenbart sind.

Ferner werden für das erfindungsgemäße Verfahren diejenigen Multimetalloxidmassen I bevorzugt eingesetzt, deren Röntgendiffraktogramm Beugungsreflexe h und i aufweist, deren Scheitelpunkte bei den Beugungswinkeln (2⊖) 22,2 ± 0,5° (h) und 27,3 ± 0,5° (i) liegen. Die Halbwertsbreite dieser Beugungsreflexe kann dabei sehr klein oder auch sehr ausgeprägt sein.

Besonders bevorzugt sind für das erfindungsgemäße Verfahren Multimetalloxidmassen I, deren Röntgendiffraktogramm zusätzlich zu den Beugungsreflexen h und i einen Beugungsreflex k aufweist, dessen Scheitelpunkt bei 28,2 ± 0,5° (k) liegt.

Unter letzteren sind wiederum jene erfindungsgemäß bevorzugt, bei denen der Beugungsreflex h innerhalb des Röntgendiffraktogramms der intensitätsstärkste ist sowie eine Halbwertsbreite von höchstens 0,5° aufweist, die Halbwertsbreite des Beugungsreflexes i und des Beugungsreflexes k gleichzeitig jeweils ≤ 1° beträgt und die Intensität $P_k$ des Beugungsreflexes k und die Intensität $P_i$ des Beugungsreflexes i die Beziehung 0,65 ≤ R ≤ 0,85 erfüllen, in der R das durch die Formel

$$R = P_i / (P_i + P_k)$$

definierte Intensitätsverhältnis ist. Bevorzugt weist das Röntgendiffraktogramm dieser Multimetalloxide I keinen Beugungsreflex auf, dessen Maximum bei 2⊖ = 50 ± 0,3° liegt.

Unter diesen Multimetalloxidmassen I sind wiederum jene für das erfindungsgemäße Verfahren vorteilhaft, für die gilt 0,67 ≤ R ≤ 0,75 und ganz besonders bevorzugt sind jene für die gilt R = 0,70 bis 0,75 bzw. R = 0,72.

Die Herstellung von Multimetalloxidmassen I, die einen Beugungsreflex i und einen Beugungsreflex k mit einer Halbwertsbreite ≤ 1° aufweisen und die gleichzeitig die Beziehung 0,65 ≤ R ≤ 0,85 erfüllen und gegebenenfalls keinen Beugungsreflex aufweisen dessen Maximum bei 2θ = 50 ± 0,3° liegt, kann wie in den Schriften DE-A 10118814 und DE-A 10119933 beschrieben erfolgen.

Es handelt sich dabei um Multimetalloxidmassen I, die ausweislich ihres Beugungsreflexes i die sogenannte i-Phase enthalten. Eine andere kristalline Phase, in der Multimetalloxidmassen I auftreten können, ist z.B. die sogenannte k-Phase. Sie weist sich aus durch das Vorhandensein der Beugungsreflexe h und k und durch ein Vorhandensein eines Beugungsreflexes mit einer Maximumslage bei 50 ± 0,3°.

Ein gezieltes Verfahren zur Herstellung von erfindungsgemäß zu verwendenden Multimetalloxidmassen I, in denen der i-Phasen Anteil dominiert, offenbaren z.B. die Schriften JP-A 11-43314, DE-A 10118814, DE-A 10119933 und die ältere Anmeldung DE-A 10046672, in welchen die relevanten Multimetalloxidmassen I als Katalysatoren für die heterogen katalysierte Oxidehydrierung von Ethan zu Ethylen sowie als Katalysatoren für die heterogen katalysierte Gasphasenoxidation von Propan bzw. Propen zu Acrylsäure empfohlen werden.

Danach wird in an sich bekannter, in den meisten zitierten Schriften des Standes der Technik offenbarter, Weise .(z.B. auch so wie in der älteren Anmeldung DE-A 10033121 beschrieben) zunächst eine Multimetalloxidmasse der Stöchiometrie (I) erzeugt, die ein Gemisch aus i-Phase und anderen Phasen (z.B. k-Phase) ist. In diesem Gemisch kann nun der Anteil an i-Phase z.B. dadurch erhöht werden, daß man die anderen Phasen, z.B. die k-Phase, unter dem Mikroskop ausliest, oder die Multimetalloxidaktivmasse mit geeigneten Flüssigkeiten wäscht. Als solche Flüssigkeiten kommen z.B. wäßrige Lösungen organischer Säuren (z.B. Oxalsäure, Ameisensäure, Essigsäure, Zitronensäure und Weinsäure), anorganischer Säuren (z.B. Salpetersäure), Alkohole und wäßrige Wasserstoffperoxidlösungen in Betracht. Desweiteren offenbart auch die JP-A 7-232071 ein Verfahren zur Herstellung von erfindungsgemäß zu verwendenden Multimetalloxidmassen I mit ausgeprägtem i-Phasen Anteil.

In weniger systematischer Weise sind erfindungsgemäß zu verwendende Multimetalloxidmassen I mit ausgeprägtem i-Phasen Anteil durch die in der DE-A 19835247 publizierte Herstellweise zugänglich. Danach wird von geeigneten Quellen ihrer elementaren Konstituenten ein möglichst inniges, vorzugsweise feinteiliges, Trockengemisch erzeugt und dieses bei Temperaturen von 350 bis 700°C bzw. 400 bis 650°C oder 400 bis 600°C thermisch behandelt. Die thermische Behandlung kann prinzipiell sowohl unter oxidierender, reduzierender als auch unter inerter Atmosphäre erfolgen. Als oxidierende Atmosphäre kommt z.B. Luft, mit molekularem Sauerstoff angereicherte Luft oder an Sauerstoff entreicherte Luft in Betracht. Vorzugsweise wird die thermische Behandlung unter inerter Atmosphäre, d.h., z.B. unter molekularem Stickstoff und/oder Edelgas, durchgeführt. Üblicherweise erfolgt die thermische Behandlung bei Normaldruck (1 atm). Selbstverständlich kann die thermische Behandlung auch unter Vakuum oder unter Überdruck erfolgen.

Erfolgt die thermische Behandlung unter gasförmiger Atmosphäre, kann diese sowohl stehen als auch fließen. Insgesamt kann die thermische Behandlung bis zu 24 h oder mehr in Anspruch nehmen.

Bevorzugt erfolgt die thermische Behandlung zunächst unter oxidierender (Sauerstoff enthaltender) Atmosphäre (z.B. unter Luft) bei einer Temperatur von 150 bis 400°C bzw. 250 bis 350°C. Im Anschluß daran wird die thermische Behandlung zweckmäßig unter Inertgas bei Temperaturen von 350 bis 700°C bzw. 400 bis 650°C oder 400 bis 600°C fortgesetzt. Selbstredend kann die thermische Behandlung auch so erfolgen, daß die Katalysatorvorläufermasse vor ihrer thermischen Behandlung zunächst (gegebenenfalls nach Pulverisierung) tablettiert (gegebenenfalls unter Zusatz von 0,5 bis 2 Gew.-% an feinteiligem Graphit), dann thermisch behandelt und nachfolgend wieder versplittet wird.

Das innige Vermischen der Ausgangsverbindungen im Rahmen der Herstellung von erfindungsgemäß zu verwendenden Multimetalloxidmassen I kann ganz generell in trockener oder in nasser Form erfolgen. Erfolgt es in trockener Form, werden die Ausgangsverbindungen zweckmäßigerweise als feinteilige Pulver eingesetzt und nach dem Mischen und gegebenenfalls Verdichten der Calcinierung (thermischen Behandlung) unterworfen.

Vorzugsweise erfolgt das innige Vermischen jedoch in nasser Form. Üblicherweise werden die Ausgangsverbindungen dabei in Form einer wäßrigen Lösung und/oder Suspension miteinander vermischt. Anschließend wird die wäßrige Masse getrocknet und nach der Trocknung calciniert. Zweckmäßigerweise handelt es sich bei der wäßrigen Masse um eine wäßrige Lösung oder um eine wäßrige Suspension. Vorzugsweise erfolgt der Trocknungsprozeß unmittelbar im Anschluß an die Herstellung der wäßrigen Mischung und durch Sprühtrocknung (die Austrittstemperaturen betragen in der Regel 100 bis 150°C; die Sprühtrocknung kann im Gleichstrom oder im Gegenstrom durchgeführt werden), die ein besonders inniges Trockengemisch bedingt, vor allem dann, wenn es sich bei der sprühzutrocknenden wäßrigen Masse um eine wäßrige Lösung handelt.

Als Quellen für die elementaren Konstituenten kommen im Rahmen der Durchführung der Herstellung von erfindungsgemäß zu verwendenden Multimetalloxidmassen I alle diejenigen in Betracht, die beim Erhitzen (gegebenenfalls an Luft) Oxide und/oder Hydroxide zu bilden vermögen. Selbstredend können als solche Ausgangsverbindungen auch bereits Oxide und/oder Hydroxide der elementaren Konstituenten mitverwendet oder ausschließlich verwendet werden.

Erfindungsgemäß geeignete Quellen für das Element Mo sind z.B. Molybdänoxide wie Molybdäntrioxid, Molybdate wie Ammoniumheptamolybdattetrahydrat und Molybdänhalogenide wie Molybdänchlorid.

Geeignete, erfindungsgemäß mitzuverwendende Ausgangsverbindungen für das Element V sind z.B. Vanadylacetylacetonat, Vanadate wie Ammoniummetavanadat, Vanadinoxide wie Vanadinpentoxid ($V_2O_5$), Vanadinhalogenide wie Vanadintetrachlorid ($VCl_4$) und Vanadinoxyhalogenide wie $VOCl_3$. Dabei können als Vanadinausgangsverbindungen auch solche mitverwendet werden, die das Vanadin in der Oxidationsstufe +4 enthalten.

Als Quellen für das Element Tellur eignen sich erfindungsgemäß Telluroxide wie Tellurdioxid, metallisches Tellur, Tellurhalogenide wie $TeCl_2$, aber auch Tellursäuren wie Orthotellursäure $H_6TeO_6$.

Vorteilhafte Antimonausgangsverbindungen sind Antimonhalogenide wie $SbCl_3$, Antimonoxide wie Antimontrioxid ($Sb_2O_3$), Antimonsäuren wie $HSb(OH)_6$, aber auch Antimonoxid-Salze wie Antimonoxid-sulfat ($(SbO)_2SO_4$).

Erfindungsgemäß geeignete Niobquellen sind z. B. Nioboxide wie Niobpentoxid ($Nb_2O_5$), Nioboxidhalogenide wie $NbOCl_3$, Niobhalogenide wie $NbCl_5$, aber auch komplexe Verbindungen aus Niob und organischen Carbonsäuren und/oder Dicarbonsäuren wie z. B. Oxalate und Alkoholate. Selbstredend kommen als Niobquelle auch die in der EP-A 895 809 verwendeten Nb enthaltenden Lösungen in Betracht.

Bezüglich aller anderen möglichen Elemente $M^2$ kommen als erfindungsgemäß geeignete Ausgangsverbindungen vor allem deren Halogenide, Nitrate, Formiate, Oxalate, Acetate, Carbonate und/oder Hydroxide in Betracht. Geeignete Ausgangsverbindungen sind vielfach auch deren Oxoverbindungen wie z. B. Wolframate bzw. die von diesen abgeleiteten Säuren. Häufig werden als Ausgangsverbindungen auch Ammoniumsalze eingesetzt.

Ferner kommen als Ausgangsverbindungen für die Erstellung der erfindungsgemäßen Multimetalloxidmassen I auch Polyanionen vom Anderson Typ in Betracht, wie sie z. B. in Polyhedron Vol. 6, No. 2, pp. 213-218, 1987 beschrieben sind. Eine weitere geeignete Literaturquelle für Polyanionen vom Anderson Typ bildet Kinetics and Catalysis, Vol. 40, No. 3, 1999, pp 401 bis 404.

Andere als Ausgangsverbindungen geeignete Polyanionen sind z. B: solche vom Dawson oder Keggin Typ. Vorzugsweise werden erfindungsgemäß solche Ausgangsverbindungen verwendet, die sich bei erhöhten Temperaturen entweder im Beisein oder bei Ausschluß von Sauerstoff, gegebenenfalls unter Freisetzung gasförmiger Verbindungen, in ihre Oxide umwandeln.

Die wie beschrieben erhältlichen erfindungsgemäß zu verwendenden Multimetalloxidmassen I können als solche [z. B. als Pulver oder nach Tablettieren des Pulvers (häufig unter Zusatz von 0,5 bis 2 Gew.-% an feinteiligem Graphit) und nachfolgendem Versplitten zu Splitt zerkleinert] oder auch zu Formkörpern geformt für das erfindungsgemäße Verfahren eingesetzt werden.

Die Formung zu Formkörpern kann z.B. durch Aufbringen auf einen Trägerkörper erfolgen, wie es in der älteren Anmeldung DE-A 10051419 beschrieben wird.

Die für die erfindungsgemäß einzusetzenden Multimetalloxidmassen I zu verwendenden Trägerkörper sind vorzugsweise chemisch inert. D.h., sie greifen in den Ablauf der katalytischen Gasphasenoxidation des Propans zu Acrylsäure, die durch die erfindungsgemäß zu verwendenden Multimetalloxidmassen katalysiert wird, im wesentlichen nicht ein.

Als Material für die Trägerkörper kommen erfindungsgemäß insbesondere Aluminiumoxid, Siliciumdioxid, Silicate wie Ton, Kaolin, Steatit, Bims, Aluminiumsilicat und Magnesiumsilicat, Siliciumcarbid, Zirkondioxid und Thoriumdioxid in Betracht.

Die Oberfläche des Trägerkörpers kann sowohl glatt als auch rauh sein. Mit Vorteil ist die Oberfläche des Trägerkörpers rauh, da eine erhöhte Oberflächenrauhigkeit in der Regel eine erhöhte Haftfestigkeit der aufgebrachten Aktivmassenschale bedingt.

Häufig liegt die Oberflächenrauhigkeit $R_z$ des Trägerkörpers im Bereich von 5 bis 200 $\mu$m, oft im Bereich von 20 bis 100 $\mu$m (bestimmt gemäß DIN 4768 Blatt 1 mit einem "Hommel Tester für DIN-ISO Oberflächenmeßgrößen" der Fa. Hommelwerke, DE).

Ferner kann das Trägermaterial porös oder unporös sein. Zweckmäßigerweise ist das Trägermaterial unporös (Gesamtvolumen der Poren auf das Volumen des Trägerkörpers bezogen $\leq$ 1 Vol.-%).

Die Dicke der auf den erfindungsgemäßen Schalenkatalysatoren befindlichen aktiven Oxidmassenschale liegt üblicherweise bei 10 bis 1000 $\mu$m. Sie kann aber auch 50 bis 700 $\mu$m, 100 bis 600 $\mu$m oder 150 bis 400 $\mu$m betragen. Mögliche Schalendicken sind auch 10 bis 500 $\mu$m, 100 bis 500 $\mu$m oder 150 bis 300 $\mu$m.

Prinzipiell kommen für das erfindungsgemäße Verfahren beliebige Geometrien der Trägerkörper in Betracht. Ihre Längstausdehnung beträgt in der Regel 1 bis 10 mm. Vorzugsweise werden jedoch Kugeln oder Zylinder, insbesondere Hohlzylinder, als Trägerkörper angewendet. Günstige Durchmesser für Trägerkugeln betragen 1,5 bis 4 mm. Werden Zylinder als Trägerkörper verwendet, so beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Erfindungsgemäß geeignete ringförmige Trägerkörper können auch eine Länge von 3 bis 6 mm, einen Außendurchmesser von 4 bis 8 mm und eine Wanddicke von 1 bis 2 mm aufweisen. Möglich ist aber auch eine Trägerringgeometrie von 7 mm x 3 mm x 4 mm oder von 5 mm x 3 mm x 2 mm (Außendurchmesser x Länge x Innendurchmesser).

Die Herstellung erfindungsgemäß zu verwendender Schalenkatalysatoren kann in einfachster Weise so erfolgen, dass man aktive Oxidmassen der allgemeinen Formel (I) vorbildet, sie in eine feinteilige Form überführt und abschließend mit Hilfe eines flüssigen Bindemittels auf die Oberfläche des Trägerkörpers aufbringt. Dazu wird die Oberfläche des Trägerkörpers in einfachster Weise mit dem flüssigen Bindemittel befeuchtet und durch Inkontaktbringen mit feinteiliger aktiver Oxidmasse der allgemeinen Formel (I) eine Schicht der Aktivmasse auf der befeuchteten Oberfläche angeheftet. Abschließend wird der beschichtete Trägerkörper getrocknet. Selbstredend kann man zur Erzielung einer erhöhten Schichtdicke den Vorgang periodisch wiederholen. In diesem Fall wird der beschichtete Grundkörper zum neuen "Trägerkörper" etc..

Die Feinheit der auf die Oberfläche des Trägerkörpers aufzubringenden katalytisch aktiven Oxidmasse der allgemeinen Formel (I) wird selbstredend an die gewünschte Schalendicke angepaßt. Für den Schalendickenbereich von 100 bis 500 μm eignen sich z. B. solche Aktivmassenpulver, von denen wenigstens 50 % der Gesamtzahl der Pulverpartikel ein Sieb der Maschenweite 1 bis 20 μm passieren und deren numerischer Anteil an Partikeln mit einer Längstausdehnung oberhalb von 50 μm weniger als 10 % beträgt. In der Regel entspricht die Verteilung der Längstausdehnungen der Pulverpartikel herstellungsbedingt einer Gaußverteilung. Häufig ist die Korngrößenverteilung wie folgt beschaffen:

| D (μm) | 1 | 1,5 | 2 | 3 | 4 | 6 | 8 | 12 | 16 | 24 | 32 |
|---|---|---|---|---|---|---|---|---|---|---|---|
| x | 80,5 | 76,3 | 67,1 | 53,4 | 41,6 | 31,7 | 23 | 13,1 | 10,8 | 7,7 | 4 |
| y | 19,5 | 23,7 | 32,9 | 46,6 | 58,4 | 68,3 | 77 | 86,9 | 89,2 | 92,3 | 96 |

| D (μm) | 48 | 64 | 96 | 128 |
|---|---|---|---|---|
| x | 2,1 | 2 | 0 | 0 |
| y | 97,9 | 98 | 100 | 100 |

Dabei sind:

D = Durchmesser des Korns,

x = der prozentuale Anteil der Körner, deren Durchmesser ≥ D ist und

y = der prozentuale Anteil der Körner, deren Durchmesser < D ist.

Für eine Durchführung des beschriebenen Beschichtungsverfahrens im technischen Maßstab empfiehlt sich z. B. die Anwendung des in der DE-A 2909671, sowie der in der DE-A 10051419 offenbarten Verfahrensprinzips. D.h., die zu beschichtenden Trägerkörper werden in einem vorzugsweise geneigten (der Neigungswinkel beträgt in der Regel ≥ 0° und ≤ 90°, meist ≥ 30° und ≤ 90°; der Neigungswinkel ist der Winkel der Drehbehältermittelachse gegen die Horizontale) rotierenden Drehbehälter (z. B. Drehteller oder Dragiertrommel) vorgelegt. Der rotierende Drehbehälter führt die z. B. kugelförmigen oder zylindrischen Trägerkörper unter zwei in bestimmtem Abstand aufeinanderfolgend angeordneten Dosiervorrichtungen hindurch. Die erste der beiden Dosiervorrichtungen entspricht zweckmäßig einer Düse (z.B. eine mit Druckluft betriebene Zerstäuberdüse), durch die die im rotierenden Drehteller rollenden Trägerkörper mit dem flüssigen Bindemittel besprüht und kontrolliert befeuchtet werden. Die zweite Dosiervorrichtung befindet sich außerhalb des Zerstäubungskegels des eingesprühten flüssigen Bindemittels und dient dazu, die feinteilige oxidische Aktivmasse zuzuführen (z.B. über eine Schüttelrinne oder eine Pulverschnecke). Die kontrolliert befeuchteten Trägerkugeln nehmen das zugeführte Aktivmassenpulver auf, das sich durch die rollende Bewegung auf der äußeren Oberfläche des z. B. zylinder- oder kugelförmigen Trägerkörpers zu einer zusammenhängenden Schale verdichtet.

Bei Bedarf passiert der so grundbeschichtete Trägerkörper im Verlauf der darauffolgenden Umdrehung wiederum die Sprühdüsen, wird dabei kontrolliert befeuchtet, um im Verlauf der Weiterbewegung eine weitere Schicht feinteiliger oxidischer Aktivmasse aufnehmen zu können usw. (eine Zwischentrocknung ist in der Regel nicht erforderlich). Feinteilige oxidische Aktivmasse und flüssiges Bindemittel werden dabei in der Regel kontinuierlich und simultan zugeführt.

Die Entfernung des flüssigen Bindemittels kann nach beendeter Beschichtung z. B. durch Einwirkung von heißen Gasen, wie $N_2$ oder Luft, erfolgen. Bemerkenswerterweise bewirkt das beschriebene Beschichtungsverfahren sowohl eine voll befriedigende Haftung der aufeinanderfolgenden Schichten aneinander, als auch der Grundschicht auf der Oberfläche des Trägerkörpers.

wesentlich für die vorstehend beschriebene Beschichtungsweise ist, dass die Befeuchtung der zu beschichtenden Oberfläche des Trägerkörpers in kontrollierter Weise vorgenommen wird. Kurz ausgedrückt heißt dies, dass man die Trägeroberfläche zweckmäßig so befeuchtet, dass diese zwar flüssiges Bindemittel adsorbiert aufweist, aber auf der Trägeroberfläche keine Flüssigphase als solche visuell in Erscheinung tritt. Ist die Trägerkörperoberfläche zu feucht, agglomeriert die feinteilige katalytisch aktive Oxidmasse zu getrennten Agglomeraten, anstatt auf die Oberfläche aufzuziehen. Detaillierte Angaben hierzu finden sich in der DE-A 2909671 und in der DE-A 10051419.

Die vorerwähnte abschließende Entfernung des verwendeten flüssigen Bindemittels kann in kontrollierter Weise z. B. durch Verdampfen und/oder Sublimieren vorgenommen werden. Im einfachsten Fall kann dies durch Einwirkung heißer Gase entsprechender Temperatur (häufig 50 bis 300, häufig 150°C) erfolgen. Durch Einwirkung heißer Gase kann aber auch nur eine Vortrocknung bewirkt werden. Die Endtrocknung kann dann beispielsweise in einem Trokkenofen beliebiger Art (z. B. Bandtrockner) oder im Reaktor erfolgen. Die einwirkende Temperatur sollte dabei nicht oberhalb der zur Herstellung der oxidischen Aktivmasse angewendeten Calcinationstemperatur liegen. Selbstverständlich kann die Trocknung auch ausschließlich in einem Trockenofen durchgeführt werden.

Als Bindemittel für den Beschichtungsprozeß können unabhängig von der Art und der Geometrie des Trägerkörpers verwendet werden: Wasser, einwertige Alkohole wie Ethanol, Methanol, Propanol und Butanol, mehrwertige Alkohole wie Ethylenglykol, 1,4-Butandiol, 1,6-Hexandiol oder Glycerin, ein- oder mehrwertige organische Carbonsäuren wie Propionsäure, Oxalsäure, Malonsäure, Glutarsäure oder Maleinsäure, Aminoalkohole wie Ethanolamin oder Diethanolamin sowie ein- oder mehrwertige organische Amide wie Formamid. Günstige Bindemittel sind auch Lösungen, bestehend aus 20 bis 90 Gew.-% Wasser und 10 bis 80 Gew.-% einer in Wasser gelösten organischen Verbindung, deren Siedepunkt oder Sublimationstemperatur bei Normaldruck (1 atm) > 100°C, vorzugsweise > 150°C, beträgt. Mit Vorteil wird die organische Verbindung aus der vorstehenden Auflistung möglicher organischer Bindemittel ausgewählt. Vorzugsweise beträgt der organische Anteil an vorgenannten wäßrigen Bindemittellösungen 10 bis 50 und besonders bevorzugt 20 bis 30 Gew.-%. Als organische Komponenten kommen dabei auch Monosaccharide und Oligosaccharide wie Glucose, Fructose, Saccharose oder Lactose sowie Polyethylenoxide und Polyacrylate in Betracht.

Von Bedeutung ist, dass die Herstellung erfindungsgemäß geeigneter Schalenkatalysatoren nicht nur durch Aufbringen der fertiggestellten, feingemahlenen aktiven Oxidmassen der allgemeinen Formel (I) auf die befeuchtete Trägerkörperoberfläche erfolgen kann.

Vielmehr kann anstelle der aktiven Oxidmasse auch eine feinteilige Vorläufermasse derselben auf die befeuchtete Trägeroberfläche (unter Anwendung der gleichen Beschichtungsverfahren und Bindemittel) aufgebracht und die Calcination nach Trocknung des beschichteten Trägerkörpers durchgeführt werden.

Als eine solche feinteilige Vorläufermasse kommt z. B. diejenige Masse in Betracht, die dadurch erhältlich ist, dass man aus den Quellen der elementaren Konstituenten der gewünschten aktiven Oxidmasse der allgemeinen Formel (I) zunächst ein möglichst inniges, vorzugsweise feinteiliges, Trockengemisch erzeugt (z. B. durch Sprühtrocknung einer wäßrigen Suspension oder Lösung der Quellen) und dieses feinteilige Trockengemisch (gegebenenfalls nach Tablettierung unter Zusatz von 0,5 bis 2 Gew.-% an feinteiligem Graphit) bei einer Temperatur von 150 bis 350°C, vorzugsweise 250 bis 350°C unter oxidierender (Sauerstoff enthaltender) Atmosphäre (z. B. unter Luft) thermisch behandelt (wenige Stunden) und abschließend bei Bedarf einer Mahlung unterwirft.

Nach der Beschichtung der Trägerkörper mit der Vorläufermasse wird dann, bevorzugt unter Inertgasatmosphäre (alle anderen Atmosphären kommen auch in Betracht) bei Temperaturen von 360 bis 700°C bzw. 400 bis 650°C oder 400 bis 600°C calciniert.

Selbstredend kann die Formgebung erfindungsgemäß verwendbarer Multimetalloxidmassen I auch durch Extrusion und/oder Tablettierung sowohl von feinteiliger Multimetalloxidmasse I, als auch von feinteiliger Vorläufermasse einer Multimetalloxidmasse I erfolgen.

Als Geometrien kommen dabei sowohl Kugeln, Vollzylinder und Hohlzylinder (Ringe) in Betracht. Die Längstausdehnung der vorgenannten Geometrien beträgt dabei in der Regel 1 bis 10 mm. Im Fall von Zylindern beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Erfindungsgemäß geeignete ringförmige Vollkatalysatoren können auch eine Länge von 3 bis 6 mm, einen Außendurchmesser von 4 bis 8 mm und eine Wanddicke von 1 bis 2 mm aufweisen. Möglich ist aber auch eine Vollkatalysatorringgeometrie von 7 mm x 3 mm x 4 mm oder von 5 mm x 3 mm x 2 mm (Außendurchmesser x Länge x Innendurchmesser).

Selbstredend kommen für das erfindungsgemäße Verfahren für die Geometrie der zu verwendenden Multimetalloxidmassen I auch all jene der DE-A 10101695 in Betracht.

Erfindungsgemäß wesentlich ist, daß erfindungsgemäß bevorzugt zu verwendenden Multimetalloxidmassen I ein Röntgendiffraktogramm aufweisen (in dieser Schrift stets bezogen auf Cu-$K_\alpha$-Strahlung), das Beugungsreflexe h, i und k aufweist, deren Scheitelpunkte bei den Beugungswinkeln (2Θ) 22,2 ± 0,4° (h), 27,3 ± 0,4° (i) und 28,2 ± 0,4° (k) liegen, wobei

- der Beugungsreflex h innerhalb des Röntgendiffraktogramms der intensitätsstärkste ist, sowie eine Halbwertsbreite von höchstens 0,5° aufweist,

- die Intensität $P_i$ des Beugungsreflexes i und die Intensität $P_k$ des Beugungsreflexes k die Bezeichnung $0{,}65 \leq R \leq 0{,}85$ erfüllen, in der R das durch die Formel

$$R = P_i / (P_i + P_k)$$

definierte Intensitätsverhältnis ist, und

- die Halbwertsbreite des Beugungsreflexes i und des Beugungsreflexes k jeweils $\leq 1°$ beträgt.

Die Definition der Intensität eines Beugungsreflexes im Röntgendiffraktogramm bezieht sich in dieser Schrift auf die in der DE-A 19835247, sowie die in der DE-A 10051419 und DE-A 10046672 niedergelegte Definition.

D.h., bezeichnet $A^1$ den Scheitelpunkt eines Reflexes 1 und bezeichnet $B^1$ in der Linie des Röntgendiffraktogramms bei Betrachtung entlang der zur 20-Achse senkrecht stehenden Intensitätsache das nächstliegende ausgeprägte Minimum (Reflexschultern ausweisende Minima bleiben unberücksichtig) links vom Scheitelpunkt $A^1$ und $B^2$ in entsprechender weise das nächstliegende ausgeprägte Minimum rechts vom Scheitelpunkt $A^1$ und bezeichnet $C^1$ den Punkt, an dem eine vom Scheitelpunkt $A^1$ senkrecht zur 20-Achse gezogene Gerade eine die Punkte $B^1$ und $B^2$ verbindende Gerade schneidet, dann ist die Intensität des Reflexes 1 die Länge des Geradenabschnitts $A^1C^1$, der sich vom Scheitelpunkt $A^1$ zum Punkt $C^1$ erstreckt. Der Ausdruck Minimum bedeutet dabei einen Punkt, an dem der Steigungsgradient einer an die Kurve in einem Basisbereich des Reflexes 1 angelegten Tangente von einem negativen Wert auf einen positiven Wert übergeht, oder einen Punkt, an dem der Steigungsgradient gegen Null geht, wobei für die Festlegung des Steigungsgradienten die Koordinaten der $2\ominus$ Achse und der Intensitätsachse herangezogen werden.

Die Halbwertsbreite ist in dieser Schrift in entsprechender Weise die Länge des Geradenabschnitts, der sich zwischen den beiden Schnittpunkten $H^1$ und $H^2$ ergibt, wenn man in der Mitte des Geradenabschnitts $A^1C^1$ eine Parallele zur $2\ominus$-Achse zieht, wobei $H^1$, $H^2$ den jeweils ersten Schnittpunkt dieser Parallelen mit der wie vorstehend definierten Linie des Röntgendiffraktogramms links und rechts von $A^1$ meinen.

Eine beispielhafte Durchführung der Bestimmung von Halbwertsbreite und Intensität zeigt auch die Figur 6 in der DE-A 10046672.

Neben den Beugungsreflexen h, i und k enthält das Röntgendiffraktogramm vorteilhafter erfindungsgemäß zu verwendender Multimetalloxidmassen I in der Regel noch weitere Beugungsreflexe, deren Scheitelpunkte bei den nachfolgenden Beugungswinkeln ($2\Theta$) liegen:

$$9{,}0 \pm 0{,}4° \ (l),$$

$$6{,}7 \pm 0{,}4° \ (o)$$

und

$$7{,}9 \pm 0{,}4° \ (p).$$

Günstig ist es, wenn das Röntgendiffraktogramm der katalytisch aktiven Oxidmassen der allgemeinen Formel (I) zusätzlich einen Beugungsreflex enthält, dessen Scheitelpunkte bei folgendem Beugungswinkel ($2\Theta$) liegt:

$$45{,}2 \pm 0{,}4° \ (q).$$

Häufig enthält das Röntgendiffraktogramm von Multimetalloxidaktivmassen (I) auch noch die Reflexe $29{,}2 \pm 0{,}4°$ (m) und $35{,}4 \pm 0{,}4°$ (n).

Enthält die katalytisch aktive Oxidmasse der allgemeinen Formel (I) k-Phase, enthält ihr Röntgendiffraktogramm in der Regel noch weiter Beugungsreflexe, deren Scheitelpunkte bei den nachfolgenden Beugungswinkeln (2Θ) liegen:

$$36{,}2 \pm 0{,}4°$$

und

$$50{,}0 \pm 0{,}4°.$$

Ordnet man dem Beugungsreflex h die Intensität 100 zu, ist es erfindungsgemäß günstig, wenn die Beugungsreflexe i, l, m, n, o, p, q in der gleichen Intensitätsskala die nachfolgenden Intensitäten aufweisen:

i: 5 bis 95, häufig 5 bis 80, teilweise 10 bis 60;

l: 1 bis 30;

m: 1 bis 40;

n: 1 bis 40;

o: 1 bis 30;

p: 1 bis 30 und

q: 5 bis 60.

Enthält das Röntgendiffraktogramm von den vorgenannten zusätzlichen Beugungsreflexen, ist die Halbwertsbreite derselben in der Regel ≤ 1°.

Alle in dieser Schrift auf ein Röntgendiffraktogramm bezogenen Angaben beziehen sich auf ein unter Anwendung von Cu-Kα-Strahlung als Röntgenstrahlung erzeugtes Röntgendiffraktogramm (Siemens-Diffraktometer Theta-Theta D-5000, Röhrenspannung: 40 kV, Röhrenstrom: 40 mA, Aperturblende V20 (variabel), Streustrahlblende V20 (variabel), Sekundärmonochromatorblende (0,1 mm), Detektorblende (0,6 mm), Meßintervall (2Θ): 0,02°, Meßzeit je Schritt: 2,4 s, Detektor: Scintillationszählrohr).

Die spezifische Oberfläche von erfindungsgemäß zu verwendenden Multimetalloxidaktivmassen (I) beträgt vielfach 1 bis 30 $m^2/g$ (BET-Oberfläche, Stickstoff).

Vorzugsweise werden beim erfindungsgemäßen verfahren frisch hergestellte erfindungsgemäß zu verwendende Katalysatoren zunächst einem Reaktionsgasausgangsgemisch ausgesetzt, das einen erhöhten Wasserdampfanteil, bezogen auf enthaltenes Propan, aufweist (d.h., im Zusammensetzungsbereich A liegt).

Wird das erfindungsgemäße Verfahren als Festbettoxidation ausgeführt, erfolgt die Durchführung in zweckmäßiger Weise in einem Rohrbündelreaktor, dessen Kontaktrohre mit dem Katalysator beschickt sind. Um die Kontaktrohre wird im Normalfall als Wärmeträger eine Flüssigkeit, in der Regel ein Salzbad geführt.

Das Reaktionsgasgemisch wird in den Kontaktrohren über den Reaktor betrachtet entweder im Gleichstrom oder im Gegenstrom zum Salzbad geführt. Das Salzbad selbst kann relativ zu den Kontaktrohren eine reine Parallelströmung ausführen. Selbstverständlich kann dieser aber auch eine Querströmung überlagert sein. Insgesamt kann das Salzbad um die Kontaktrohre auch eine mäanderförmige Strömung ausführen, die nur über den Reaktor betrachtet im Gleich- oder im Gegenstrom zum Reaktionsgasgemisch geführt ist. Für das erfindungsgemäße Verfahren geeignete Rohrbündelreaktoren offenbaren z.B. die Schriften EP-A 700714 und EP-A 700893. Die Belastung der Katalysatorbeschickung mit Propan kann beim erfindungsgemäßen Verfahren z.B. 10 bis 500 Nl/l·h betragen. Die Belastung mit Reaktionsgasausgangsgemisch liegt häufig im Bereich von 100 bis 10000 Nl/l·h, vielfach im Bereich 500 bis 5000 Nl/l·h.

Das Reaktionsgasausgangsgemisch kann beim erfindungsgemäßen Verfahren der die Katalysatoren enthaltenden Reaktionszone auf die Reaktionstemperatur vorerwärmt zugeführt werden.

Selbstredend wird beim erfindungsgemäßen Verfahren ein Produktgasgemisch erhalten, das nicht ausschließlich aus Acrylsäure besteht. Vielmehr enthält das Produktgasgemisch neben nicht umgesetztem Propan Nebenkomponenten wie Propen, Acrolein, $CO_2$, CO, $H_2O$, Essigsäure, Propionsäure etc., von denen die Acrylsäure abgetrennt werden muß.

Da durch die erfindungsgemäße Verfahrensweise sowohl die im Produktgasgemisch enthaltene Menge an Wasserdampf als auch die Nebenproduktbildung an Essig- und Propionsäure beschränkt wird, kann diese Abtrennung so erfolgen, wie es von der heterogen katalysierten Gasphasenoxidation von Propen zu Acrylsäure allgemein bekannt ist.

D.h., aus dem Produktgasgemisch kann die enthaltene Acrylsäure z.B. durch die Absorption mit einem hochsiedenden inerten hydrophoben organischen Lösungsmittel (z.B. einem Gemisch aus Diphenylether und Diphyl das gegebenenfalls noch Zusätze wie Dimethylphthalat enthalten kann) aufgenommen werden. Das dabei resultierende Gemisch aus Absorbens und Acrylsäure kann anschließend in an sich bekannter Weise rektifikativ, extraktiv und/oder kristallisativ bis zur Reinacrylsäure aufgearbeitet werden. Alternativ kann die Grundabtrennung der Acrylsäure aus dem Produktgasgemisch auch durch fraktionierte Kondensation erfolgen, wie es z.B. in der DE-A 19924532 beschrieben ist.

Das dabei resultierende Acrylsäurekondensat kann dann z.B. durch fraktionierte Kristallisation (z.B. Suspensionskristallisation und/oder Schichtkristallisation) weitergereinigt werden.

Das bei der Grundabtrennung der Acrylsäure verbleibende Restgasgemisch enthält insbesondere nicht umgesetztes Propan und gegebenenfalls nicht umgesetztes Propen. Dieses kann aus dem Restgasgemisch z.B. durch fraktionierte Druckrektifikation abgetrennt und anschließend in die erfindungsgemäße Gasphasenoxidation rückgeführt werden. Günstiger ist es jedoch, das Restgas in einer Extraktionsvorrichtung mit einem hydrophoben organischen Lösungsmittel in Kontakt zu bringen (z.B. durch selbiges durchleiten), das das Propan und gegebenenfalls Propen bevorzugt zu absorbieren vermag.

Durch nachfolgende Desorption und/oder Strippung mit Luft kann das absorbierte Propan und gegebenenfalls Propen wieder freigesetzt und in das erfindungsgemäße Verfahren rückgeführt werden. Auf diese Weise sind wirtschaftliche Gesamtpropanumsätze erzielbar. Selbstverständlich kann die Acrylsäure aber auch nach der in der DE-A 10059122 beschriebenen Verfahrensweise aus dem Produktgasgemisch abgetrennt werden.

Bemerkenswert am erfindungsgemäßen Verfahren ist, daß es bei vermindertem Wasserdampfbedarf hohe Selektivitäten der Acrylsäurebildung ermöglicht.

Selbstredend können die erfindungsgemäß zu verwendenden Multimetalloxidmassen I auch in mit feinteiligen, z.B. kolloidalen, Materialien wie Siliciumdioxid, Titandioxid, Aluminiumoxid, Zirkonoxid und Nioboxid verdünnter Form im erfindungsgemäßen Verfahren eingesetzt werden.

Das Verdünnungsmassenverhältnis kann dabei bis zu 9 (Verdünner) : 1 (Aktivmasse) betragen. D.h., mögliche Verdünnungsmassenverhältnisse betragen z.B. 6 (Verdünner) : 1 (Aktivmasse) und 3 (Verdünner) : 1 (Aktivmasse). Die Einarbeitung der Verdünner kann vor und/oder nach der Calcination erfolgen. Erfolgt die Einarbeitung vor der Calcination, muß der Verdünner so gewählt werden, daß er bei der Calcination als solches im wesentlichen erhalten bleibt. Dies ist z.B. im Fall von bei entsprechend hohen Temperaturen gebrannten Oxiden in der Regel gegeben.

Beim erfindungsgemäßen Verfahren verbrauchte Katalysatoren können durch Beschicken mit sauerstoffhaltigen Gasen, z.B. Luft oder an Sauerstoff entreicherte oder angereicherte Luft, denen auch Wasserdampf zugesetzt sein kann, bei Temperaturen ≤ Reaktionstemperatur mehrfach regeneriert werden.

Beispiele

A) Herstellung eines erfindungsgemäß zu verwendenden Multimetalloxid-I-Katalysators

In 44,6 1 Wasser wurden bei 80°C in einem Edelstahlbehälter unter Rühren 1287,25 g Ammoniummetavanadat (77,5 Gew.-% $V_2O_5$, Fa. G.f.E., DE-Nürnberg) gelöst. Es entstand eine klare gelbliche Lösung. Diese Lösung wurde auf 60°C abgekühlt und dann nacheinander in der genannten Reihenfolge unter Aufrechterhaltung der 60°C zunächst 1683,75 g Tellursäure (99 Gew.-% $H_6TeO_6$, Fa. Fluka) und dann 5868,0 g Ammoniumheptamolybdat (81,5 Gew.-% $MoO_3$, Fa. Starck) eingerührt. Es entstand eine tiefrote Lösung A. In einem zweiten Edelstahlbehälter wurden bei 60°C in 8,3 1 Wasser 1599,0 g Ammoniumnioboxalat (21,1 Gew.-% Nb, Fa. Starck, DE-Goslar) zu einer Lösung B gelöst. Die beiden Lösungen A und B wurden auf 30°C abgekühlt und bei dieser Temperatur miteinander vereinigt, wobei die Lösung B in die Lösung A eingerührt wurde. Das Einrühren erfolgte kontinuierlich innerhalb eines Zeitraums von 10 Minuten. Es entstand eine orange gefärbte wäßrige Suspension.

Diese Suspension wurde anschließend sprühgetrocknet ($T_{Vorlagebehälter}$ = 30°C, $T^{ein}$ = 240°C, $T^{aus}$ = 110°C, Trocknungsdauer: 1,5 h, Sprühturm der Fa. Nipolosa). Das anfallende Sprühgut war ebenfalls orange gefärbt. In das Sprühgut wurde 1 Gew.-% feinteiliges Graphit (Siebanlayse: min. 50 Gew.-% < 24 μm, max. 10 Gew.-% > 24 μm und < 48 μm, max. 5 Gew.-% > 48 μm, BET-Oberfläche: 6 bis 13 $m^2$/g) eingemischt.

Das resultierende Gemisch wurde zu Hohlzylindern (Ringen) der Geometrie 16 mm x 2,5 mm x 8 mm (Außendurchmesser x Höhe x Innendurchmesser) so kompaktiert (verpreßt), daß die resultierende Seitendruckfestigkeit der Ringe ca. 10 N betrug.

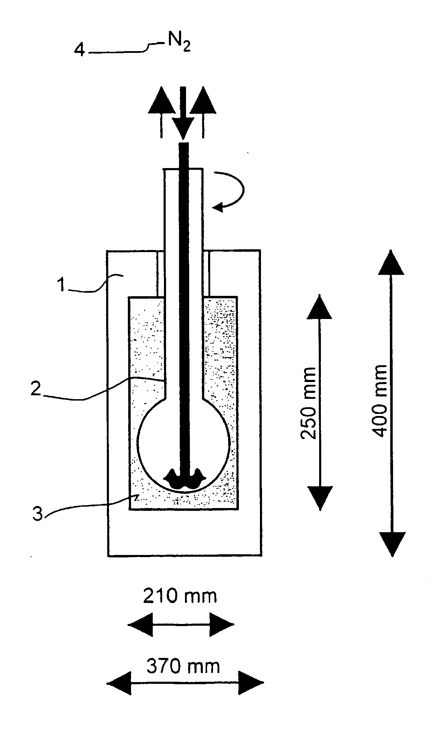

200 g dieser Ringe wurden in zwei Portionen zu jeweils 100 g nacheinander in einem Drehkugelofen gemäß Figur 1 calciniert (Quarzglaskugel mit 1 Liter Innenvolumen; 1 = Ofengehäuse, 2 = Drehkolben, 3 = beheizter Raum, 4 = Stickstoff-/Luftstrom). Dazu wurde der Drehkugelofeninhalt unter einem Luftstrom von 50 Nl/h mit linearer Heizrampe innerhalb von 27,5 min. von 25°C auf 275°C aufgeheizt und bei dieser Temperatur unter Aufrechterhaltung des Luftstroms für 1 h gehalten. Anschließend wurde innerhalb von 32,5 min. mit linearer Heizrampe von 275°C auf 600°C aufgeheizt, wobei der Luftstrom durch einen Stickstoffstrom von 50 Nl/l ersetzt wurde. Die 600°C und der Stickstoffstrom wurden für 2 h aufrechterhalten und der gesamte Ofen anschließend durch sich selbst überlassen unter Aufrechterhaltung des Stickstoffstroms auf 25°C abkühlen lassen. Es resultierten schwarze Tabletten der Zusammensetzung $Mo_{1,0}V_{0,33}Te_{0,15}Nb_{0,11}O_x$.

In einer Retsch-Mühle wurden die Tabletten trocken auf eine Korngröße < 100 μm aufgemahlen. 150 g des aufgemahlenen Materials wurden in 1500 ml einer 10 gew.-%igen wäßrigen $HNO_3$-Lösung während 7 h bei 70°C unter Rückfluß gerührt, der Feststoff aus der resultierenden Aufschlämmung abfiltriert und mit Wasser nitratfrei gewaschen. Der Filterkuchen wurde über Nacht unter Luft bei 110°C in einem Muffelofen getrocknet. Die resultierende Aktivmasse hatte die Zusammensetzung $Mo_{1,0}V_{0,27}Te_{0,12}Nb_{0,13}O_x$.

75 g des erhaltenen Aktivmassenpulvers wurden auf 300 g kugelförmige Trägerkörper mit einem Durchmesser von 2,2 - 3,2 mm (Trägermaterial = Steatit der Fa. Ceramtec, DE, Porengesamtvolumen des Trägers ≤ 1 Vol.-% bezogen auf das Trägergesamtvolumen; $R_z$ = 45 μm) aufgebracht. Dazu wurden die Trägerkörper in einer Dragiertrommel mit 2 1 Innenvolumen (Neigungswinkel der Trommelmittelachse gegen die Horizontale = 30°) vorgelegt. Die Trommel wurde mit 25 Umdrehungen je Minute in Rotation versetzt. Über eine mit 300 Nl/h Druckluft betriebene Zerstäuberdüse wurden über 60 min. hinweg ca. 30 ml eines Gemisches von Glycerin und Wasser (Gewichtsverhältnis Glycerin: Wasser = 1: 3) auf die Trägerkörper gesprüht. Die Düse war dabei derart installiert, daß der Sprühkegel die in der Trommel durch Mitnahmebleche an den obersten Punkt der geneigten Trommel beförderten Trägerkörper in der oberen Hälfte der Abrollstrecke benetzte. Das feinteilige Aktivmassenpulver wurde über eine Pulverschnecke in die Trommel eingetragen, wobei der Punkt der Pulverzugabe innerhalb der Abrollstrecke aber unterhalb des Sprühkegels lag. Durch die periodische Wiederholung von Benetzung und Pulveraufdosierung wurde der grundbeschichtete Trägerkörper in der darauffolgenden Periode selbst zum Trägerkörper.

Nach Abschluß der Beschichtung wurden die beschichteten Trägerkörper während 16 Stunden bei 120°C im Umlufttrockenschrank (Firma Binder, DE, Innenvolumen 53 1) getrocknet. Glycerin wurde durch eine sich daran anschließende zweistündige Wärmebehandlung bei 150°C unter Luft entfernt. Es wurde ein erfindungsgemäß zu verwendender Schalenkatalysator S mit 20 Gew.-% Aktivmassenanteil erhalten.

B) Durchführung des erfindungsgemäßen Verfahrens und eines Vergleichsverfahrens

Jeweils 35 g von frisch hergestelltem Schalenkatalysator S wurden in einen Einzelrohrreaktor eingebaut (Rohrlänge: 140 cm, Innendurchmesser: 8,5 mm, Außendurchmesser: 60 mm, V2A Stahl, Katalysatorschüttlänge: 54,5 cm, zusätzlich zum Anwärmen des Reaktionsgasausgangsgemischs eine 30 cm lange Vorschüttung aus Steatitkugeln der Fa. Ceramtec (2,2 - 3,2 mm Durchmesser), weiterhin war das Reaktionsrohr mit den selben Steatitkugeln nach der Katalysatorzone abschließend aufgefüllt), der durch elektrische Heizmatten beheizt wurde. Bei einer Mattentemperatur von 350°C wurde der Schalenkatalysator an Luft in den Rohrreaktor eingebaut.

Danach wurden in vier voneinander unabhängigen versuchen W, X, Y und Z vier frische Rohrreaktorkatalysatorfüllungen während 48 h bei einer Heizmattentemperatur von 350°C mit den nachfolgenden Reaktionsgasausgangsgemischen W, X, Y und Z beschickt:

W: 3,3 Vol.-% Propan, 10 Vol.-% $O_2$, 40 Vol.-% $N_2$, 46,7 Vol.-% $H_2O$;
X: 3,3 Vol.-% Propan, 10 Vol.-% $O_2$, 40 Vol.-% $N_2$, 46,7 Vol.-% $H_2O$;
Y: 3,3 Vol.-% Propan, 10 Vol.-% $O_2$, 70 Vol.-% $N_2$, 16,7 Vol.-% $H_2O$;
Z: 3,3 Vol.-% Propan, 10 Vol.-% $O_2$, 86,7 Vol.-% $N_2$, 0 Vol.-% $H_2O$.

Die Verweilzeit (bezogen auf das Katalysatorschüttvolumen) betrug in allen Fällen 2,4 s. Der Arbeitsdruck betrug in allen Fällen 2 bar absolut.

Im Abschluß an die 48 h wurden die Reaktionsrohre unter Beibehalt der Verweilzeit mit den nachfolgenden Reaktionsgasausgangsgemischzusammensetzungen weiter beschickt und dabei nach einer weiteren Betriebsdauer von 4 h die nachfolgenden Ergebnisse (bei einfachem Reaktionsrohrdurchgang) erzielt ($T_M$ = angewandte Heizmattentemperatur):

W (erfindungsgemäß): 3,3 Vol.-% Propan, 10 Vol.-% $O_2$, 86,7 Vol.-%
$N_2$, 0 Vol.-% $H_2O$,
$T_M$ = 390°C,
Umsatz an Propan bei einmaligem Reaktionsrohrdurchgang ($U_p$): 25 mol-%,

(fortgesetzt)

|  | Selektivität $S_{AS}$ der Acrylsäurebildung bei einmaligem Reaktionsrohrdurchgang: 50 mol-%. |
|---|---|
| Z (Vergleich zu W): | 3,3 Vol.-% Propan, 10 Vol.-% $O_2$, 86,7 Vol.-% $N_2$, 0 Vol.-% $H_2O$, $T_M$ = 390°C, $U_p$ = 25 mol-%, $S_{AS}$ = 40 mol-%. |
| X (erfindungsgemäß): | 3,3 Vol.-% Propan, 10 Vol.-% $O_2$, 70 Vol.-% $N_2$, 16,7 Vol.-% $H_2O$, $T_M$ = 370°C, $U_p$ = 25 mol-%, $S_{AS}$ = 70 mol-%. |
| Y (vergleich zu X): | 3,3 Vol.-% Propan, 10 Vol.-% $O_2$, 70 Vol.-% $N_2$, 16,7 Vol.-% $H_2O$, $T_M$ = 385°C, $U_p$ = 25 mol-%, $S_{AS}$ = 50 mol-%. |

Die erzielten Ergebnisse weisen die Vorteilhaftigkeit der erfindungsgemäßen Verfahrensweise aus.

**Patentansprüche**

1. Verfahren zur Herstellung von Acrylsäure durch heterogen katalysierte Partialoxidation von Propan in der Gasphase, bei dem man ein Propan, molekularen Sauerstoff und wenigstens ein Verdünnungsgas enthaltendes Reaktionsgasausgangsgemisch bei erhöhter Temperatur über eine Multimetalloxidmasse der allgemeinen Stöchiometrie I

$$Mo_1V_bM^1_cM^2_dO_n \qquad (I),$$

mit

M$^1$ = Te und/oder Sb,
M$^2$ = wenigstens eines der Elemente aus der Gruppe umfas- send Nb, Ta, W, Ti, Al, Zr, Cr, Mn, Ga, Fe, Ru, Co, Rh, Ni, Pd, Pt, La, Bi, B, Ce, Sn, Zn, Si und In,
b = 0,01 bis 1,
c = > 0 bis 1,
d = > 0 bis 1 und
n = eine Zahl, die durch die wertigkeit und Häufigkeit der von Sauerstoff verschiedenen Elemente in (I) bestimmt wird,

leitet und dabei das Propan partiell zu Acrylsäure oxidiert, **dadurch gekennzeichnet, daß** die Zusammensetzung des Reaktionsgasausgangsgemisches während der Durchführung des Verfahrens wenigstens einmal so geändert wird, daß der im Reaktionsgasausgangsgemisch, bezogen auf die im Reaktionsgasausgangsgemisch enthaltene molare Menge an Propan, vor der Änderung enthaltene Anteil des Verdünnungsgases Wasserdampf nach der Änderung ein geringerer ist.

2. Verfahren nach Anspruch 1, **dadurch gekennzeichnet, daß** im Reaktionsgasausgangsgemisch vor der Änderung seiner Zusammensetzung folgende molaren Verhältnisse vorliegen: Propan:Sauerstoff:$H_2O$:sonstige Verdünnungsgase = 1:(0,1-10):(0,1-50):(0-50).

3. Verfahren nach Anspruch 1 oder 2, **dadurch gekennzeichnet, daß** im Reaktionsgasausgangsgemisch nach der Änderung seiner Zusammensetzung folgende molaren Verhältnisse vorliegen: Propan:Sauerstöff:$H_2O$:sonstige Verdünnungsgase = 1:(0,1-10):(0-30):(0-30).

4. Verfahren nach einem der Ansprüche 1 bis 3, **dadurch gekennzeichnet, daß** die Reaktionstemperatur vor der Änderung der Zusammensetzung des Reaktionsgasausgangsgemisches 250 bis 550°C beträgt.

**Claims**

1. A process for the preparation of acrylic acid by heterogeneously catalyzed partial oxidation of propane in the gas phase, in which a reaction gas starting mixture comprising propane, molecular oxygen and at least one diluent gas is passed at elevated temperatures over a multimetal oxide material having the stoichiometry I

$$Mo_1V_bM^1_cM^2_dO_n \qquad (I),$$

where

$M^1$ is Te and/or Sb,
$M^2$ is at least one of the elemens from the group consisting of Nb, Ta, W, Ti, Al, Zr, Cr, Mn, Ga, Fe, Ru, Co, Rh, Ni, Pd, Pt, La, Bi, B, Ce, Sn, Zn, Si and In,
b is from 0.01 to 1,
c is from > 0 to 1,
d is from > 0 to 1 and
n is a number which is determined by the valency and frequency of the elements other than oxygen in (I),

and the propane is thereby partially oxidized to acrylic acid, wherein the composition of the reaction gas starting mixture is changed at least once while the process is being carried out, in such a way that the proportion of the diluent gas steam present in the reaction gas starting mixture before the change and relative to the molar amount of propane present in the reaction gas starting mixture is lower after the change.

2. The process according to claim 1, wherein the following molar ratios are present in the reaction gas starting mixture before its composition is changed:

propane:oxygen:$H_2O$:other diluent gases = 1:(0.1-10):(0.1-50):(0-50).

3. The process according to claim 1 or 2, wherein the following molar ratios are present in the reaction gas starting mixture after its composition has been changed:

propane:oxygen:$H_2O$:other diluent gases = 1:(0.1-10):(0-30):(0-30).

4. The process according to any of claims 1 to 3, wherein the reaction temperature before the composition of the reaction gas starting mixture is changed is from 250 to 550°C.

**Revendications**

1. Procédé pour la préparation d'acide acrylique par oxydation partielle catalysée par voie hétérogène de propane en phase gazeuse, dans lequel on fait passer un mélange de départ de gaz réactionnel contenant du propane, de l'oxygène moléculaire et au moins un gaz de dilution, à température élevée, sur une masse d'oxyde multimétallique de stoechiométrique I

$$Mo_1V_bM^1_cM^2_dO_n \qquad (I),$$

avec

$M^1$ = Te et/ou Sb,
$M^2$ = au moins un des éléments du groupe comprenant Nb, Ta, W, Ti, Al, Zr, Cr, Mn, Ga, Fe, Ru, Co, Rh, Ni, Pd, Pt, La, Bi, B, Ce, Sn, Zn, Si et In,
b = 0,01 à 1,
c = > 0 à 1,
d = > 0 à 1 et

n = un nombre qui est déterminé par la valence et la fréquence des éléments différents de l'oxygène dans (I),

et on oxyde ce faisant le propane partiellement en acide acrylique, **caractérisé en ce que** la composition du mélange de départ de gaz réactionnel est modifiée au moins une fois pendant la réalisation du procédé de manière telle que la proportion contenue avant la modification dans le mélange de départ de gaz réactionnel, par rapport à la quantité molaire de propane contenue dans le mélange de départ de gaz réactionnel, de gaz de dilution, la vapeur d'eau, est plus faible après la modification.

2. Procédé selon la revendication 1, **caractérisé en ce qu'**il existe dans le mélange de départ de gaz réactionnel, avant la modification de sa composition, les rapports molaires suivants : propane:oxygène:$H_2O$:autres gaz de dilution = 1:(0,1-10):(0,1-50):(0-50).

3. Procédé selon la revendication 1 ou 2, **caractérisé en ce qu'**il existe dans le mélange de départ de gaz réactionnel, après la modification de sa composition, les rapports molaires suivants : propane:oxygène:$H_2O$:autres gaz de dilution = 1:(0,1-10):(0-30):(0-30).

4. Procédé selon l'une quelconque des revendications 1 à 3, **caractérisé en ce que** la température de réaction avant la modification de la composition du mélange de départ de gaz réactionnel, est de 250 à 550°C.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10119933 A
- DE 10051419 A
- DE 10046672 A
- DE 10033121 A
- EP 1090684 A
- EP 962253 A
- EP 895809 A
- DE 19835247 A
- EP 608838 A
- WO 0029105 A
- WO 0029106 A

- EP 608838 B
- DE 10118814 A
- JP 11043314 A
- JP 7232071 A
- DE 2909671 A
- DE 10101695 A
- EP 700714 A
- EP 700893 A
- DE 19924532 A
- DE 10059122 A

**In der Beschreibung aufgeführte Nicht-Patentliteratur**

- *Polyhedron,* 1987, vol. 6 (2), 213-218

- *Anderson Typ bildet Kinetics and Catalysis,* 1999, vol. 40 (3), 401-404